(19) Europäisches Patentamt European Patent Office Office européen des brevets

(11) **EP 4 606 293 A1**

(12) **EUROPEAN PATENT APPLICATION**

(43) Date of publication:
**27.08.2025   Bulletin 2025/35**

(21) Application number: **24159282.3**

(22) Date of filing: **23.02.2024**

(51) International Patent Classification (IPC):
**A61B 1/00** (2006.01)     **A61B 1/005** (2006.01)

(52) Cooperative Patent Classification (CPC):
**A61B 1/000094; A61B 1/00006; A61B 1/000096; A61B 1/0016; A61B 1/0051**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Medintech Inc.**
**Seoul 03100 (KR)**

(72) Inventors:
• **LEE, Chi Won**
  **Namyangju-si, Gyeonggi-do (KR)**
• **KIM, Myung Joon**
  **Gwacheon-si, Gyeonggi-do (KR)**
• **KIM, Tae Hyeon**
  **Seoul (KR)**
• **CHO, Seok Ho**
  **Seoul (KR)**
• **KIM, Gun O**
  **Seoul (KR)**
• **KIM, Dong Hyeok**
  **Seoul (KR)**

(74) Representative: **Gualeni, Nadia**
**Jacobacci & Partners S.p.A.**
**Piazza della Vittoria, 11**
**25122 Brescia (IT)**

(54) **ENDOSCOPIC DEVICE AND CONTROL METHOD FOR ACQUIRING UPPER GASTROINTESTINAL TRACT IMAGES**

(57)     Provided are a medical image device and an automated control technique, particularly, an endoscopic device (100) for capturing an image of an upper gastrointestinal tract using an endoscope and a method of controlling the endoscopic device. The method of controlling an endoscopic device includes acquiring an image of an upper gastrointestinal tract from an image sensor (151), detecting at least one first body part from the image, based on a pre-trained model, calculating relative position information between the detected at least one first body part and a front-end portion of the endoscopic device, generating, based on the relative position information, a first control signal for steering the front-end portion to correspond to the at least one first body part, and transmitting the first control signal to a driver (130).

FIG. 2

EP 4 606 293 A1

## Description

[BACKGROUND]

Provided are a medical image device and an automated control technique, particularly, an endoscopic device for capturing an image of an upper gastrointestinal tract using an endoscope and a method of controlling the endoscopic device.

[DESCRIPTION OF THE RELATED ART]

An endoscope generally refers to a medical device for inserting a scope into a human body and observing an organ without surgery or autopsy. An endoscope may be used to insert a scope into a human body, irradiate light, and visualize light reflected from a surface of an inner wall in the human body. Depending on the purpose and human body part, endoscopes are classified into two main types: rigid endoscopes having a scope including a metal and flexible endoscopes represented by gastrointestinal endoscopes.

Nowadays, when an endoscopic specialist discovers a lesion during an endoscopic treatment, a biopsy has to be performed by inserting an instrument into the human body or an additional operation such as pressing a button of the scope, etc. has to be performed. In this situation, when the endoscopic specialist lets go of the scope in his/her hand, the scope may be shaken and the lesion may get out of an image view.

Such an endoscopic technique mainly depends on manual manipulation by a medical specialist to adjust a front end of an endoscope and acquire an image of an internal body part of a patient. This process is highly dependent upon the level of skills and the experience of the medical specialist, and thus, it may be difficult to obtain a precise and clear image of the body part. In particular, due to an unnecessary motion or difficulty in precise adjusting during the process of manipulating the endoscope, an image of a certain part required for an accurate diagnosis may not be sufficiently obtained.

Also, the endoscopic technique has limitations for precisely adjusting a position and an angle of the front end of the endoscope, and thus, acquiring an image of a part having many curves, such as an upper gastrointestinal tract, may be difficult. These limitations may deteriorate the efficiency and accuracy of the endoscopic diagnosis for early discovery of a disease and identification of a precise position of the disease.

[CONTENT OF THE DISCLOSURE]

[PROBLEM TO BE SOLVED]

Provided are a method and a device for controlling, based on an artificial intelligence (AI) neural network, a position and orientation of a front end of an endoscope.

[MEANS TO SOLVE THE PROBLEM]

According to an aspect of the disclosure, a method of controlling an endoscopic device includes acquiring an image of an upper gastrointestinal tract from an image sensor, detecting at least one first body part from the image, based on a pre-trained model, calculating relative position information between the detected at least one first body part and a front-end portion of the endoscopic device, generating, based on the relative position information, a first control signal for steering the front-end portion to correspond to the at least one first body part, and transmitting the first control signal to a driver.

The acquiring of the image of the upper gastrointestinal tract may include acquiring a first image at a first point and acquiring a second image at a second point. Also, the calculating of the relative position information may include calculating a target rotation angle of the front-end portion of the endoscopic device, based on i) an angle change of the front-end portion of the endoscopic device between the first point and the second point and ii) a change between the first image and the second image.

The method may further include identifying at least one second body part from the image, based on the pre-trained model, calculating relative pose information between the identified at least one second body part and the front-end portion, generating, based on the relative pose information, a second control signal with respect to photographing of the identified at least one second body part, and transmitting the second control signal to the driver.

The calculating of the relative pose information may include identifying, based on the acquired image and brightness information with respect to an illuminator, a brightness difference on the image, estimating, based on the identified brightness difference, a distance between the front-end portion and the at least one second body part, and calculating the relative pose information, based on the estimated distance.

The acquiring of the image of the upper gastrointestinal tract may include acquiring a first image at a first point and

acquiring a second image at a second point. Also, the calculating of the relative pose information may include estimating a distance between the front-end portion and the at least one second body part, based on i) an angle change of the front-end portion between the first point and the second point and ii) a change between the first image and the second image and calculating the relative pose information.

The pre-trained model may include a classification model and a detection model both trained by using, as a data set, an image labelled with respect to the at least one first body part and the at least one second body part with respect to the upper gastrointestinal tract.

The generating of the second control signal may include identifying at least one photographing point corresponding to the identified at least one second body part, generating the second control signal, based on the at least one photographing point and the relative position information, and photographing an image when a position of the front-end portion corresponds to the at least one photographing point.

The method may further include adjusting a tension of at least one wire connected to the driver and controlling a rotation angle of the front-end portion, based on the first control signal.

The method may further include generating torque feedback and transmitting the torque feedback to a manipulator, via the driver.

The torque feedback may have a positive correlation with a target rotation angle for moving the front-end portion according to the first control signal with respect to the photographing of the image.

According to another aspect of the disclosure, an endoscopic device includes a front-end portion including an image sensor, a driver configured to control a rotation angle of the front-end portion, and a controller configured to detect, based on a pre-trained model, at least one first body part from an image, calculate relative position information between the detected at least one first body part and the front-end portion, generate, based on the relative position information, a first control signal for steering the front-end portion to correspond to the at least one first body part, and transmit the first control signal to the driver.

The controller may further be configured to acquire a first image at a first point and a second image at a second point and calculate, based on i) an angle change of the front-end portion between the first point and the second point and ii) a change between the first image and the second image, a target rotation angle of the front-end portion.

The controller may further be configured to identify, based on the pre-trained model, at least one second body part from the image, calculate relative pose information between the identified at least one second body part and the front-end portion, generate, based on the relative pose information, a second control signal with respect to photographing of the at least one second body part, and transmit the second control signal to the driver.

The controller may further be configured to identify, based on the acquired image and brightness information with respect to an illuminator, a brightness difference on the image, estimate, based on the identified brightness difference, a distance between the front-end portion and the at least one second body part, and calculate the relative pose information, based on the estimated distance.

The controller may further be configured to acquire a first image at a first point and a second image at a second point, and based on i) an angle change of the front-end portion between the first point and the second point and ii) a change between the first image and the second image, estimate a distance between the front-end portion and the at least one second body part and calculate the relative pose information.

The pre-trained model may include a classification model and a detection model both trained by using, as a data set, an image labelled with respect to the at least one first body part and the at least one second body part with respect to the upper gastrointestinal tract.

The controller may further be configured to identify at least one photographing point corresponding to the identified at least one second body part, generate the second control signal, based on the at least one photographing point and the relative position information, and photograph an image when a position of the front-end portion corresponds to the at least one photographing point.

The endoscopic device may further include a curved portion connected to the front-end portion, and the controller may further be configured to control the driver based on the first control signal and adjust a tension of at least one wire connected to the driver so as to adjust, based on a curve of the curved portion, the rotation angle of the front-end portion.

The endoscopic device may further include a manipulator including a curved steering portion, and the controller may further be configured to generate torque feedback and transmit the torque feedback to the curved steering portion, via the driver.

The torque feedback may have a positive correlation with a target rotation angle for moving the front-end portion according to the first control signal with respect to the photographing of the image.

[EFFECTS OF THE DISCLOSURE]

According to an embodiment, a controller of an endoscopic device may control a position of a front-end portion, and thus, an image of a certain body part may be accurately and efficiently acquired. Thus, the degree of difficulty of endoscopic manipulation may be decreased, and the accuracy of medical diagnosis may be increased.

Effects of the one or more of the embodiments described above are not limited to the effects described above, and other effects that not are described may be clearly understood by one of ordinary skill in the art from the disclosure of the claims.

[BRIEF DESCRIPTION OF THE DRAWINGS]

FIG. 1 schematically illustrates an endoscopic device according to an embodiment.
FIG. 2 is a view for describing a process of controlling a curved portion by a driver and wires, according to an embodiment.
FIG. 3 is a flowchart of an operation of generating a learning model, according to an embodiment.
FIG. 4A is a flowchart of an operation of an endoscopic device, according to an embodiment.
FIG. 4B is a flowchart of an operation of an endoscopic device, according to another embodiment.
FIG. 4C is a flowchart of an operation of an endoscopic device, according to another embodiment.
FIG. 5 is a flowchart of an operation, performed by an endoscopic device, of calculating relative pose information, according to an embodiment.
FIG. 6 is a flowchart of an operation, performed by an endoscopic device, of calculating relative pose information, according to another embodiment.
FIG. 7 is a flowchart of an operation, performed by an endoscopic device, of calculating relative pose information, according to another embodiment.
FIG. 8 is a flowchart of an operation, performed by an endoscopic device, of capturing an image, according to an embodiment.
FIG. 9 is a flowchart of an operation of an endoscopic device with respect to torque feedback, according to an embodiment.
FIG. 10 is a block diagram of a computer device according to an embodiment.

[DETAILED DESCRIPTION]

Terms used in the disclosure are only for describing particular embodiments and may not be intended to limit the scope of other embodiments. A singular expression may include a plural expression, unless an apparently different meaning is indicated in the context. Terms used herein including technical or scientific terms may have the meanings that are the same as the meanings commonly understood by one of ordinary skill in the art described in the disclosure. Terms defined in general dictionaries from among terms used in the disclosure may be interpreted to have the meanings that are the same or substantially the same as the meanings in the context of the art and may not be interpreted to have ideal or excessively formal meanings unless overtly defined so in the disclosure. According to cases, terms may not be interpreted to exclude embodiments, even if the terms are defined in the disclosure.

Hereinafter, various embodiments are described in detail with reference to the accompanying drawings for one of ordinary skill in the art to easily execute the embodiments. However, the technical concept of the disclosure may be embodied in various changed forms, and thus, the disclosure is not limited to the embodiments described in this specification. When describing the embodiments in this specification, descriptions of the related well-known art may be omitted, when those descriptions are determined to have the possibility of obscuring the gist of the technical concept of the disclosure. The same or substantially the same elements are given the same reference numerals, and the same descriptions thereof are not repeated.

Here, the term "~ unit" used in the embodiments refers to a component performing certain functions performed by software or hardware such as a field-programmable gate array (FPGA) or an application-specific integrated circuit (ASIC). However, the term "~ unit" is not limited to the component performing certain functions performed by software or hardware. The term "-unit" may be in the form of data stored in a storage medium that may be addressed, or may be realized by an instruction for one or more processors to perform certain functions.

Software may include a computer program, a code, an instruction, or a combination of at least two thereof, and may configure a processing device or independently or collectively instruct the processing device to operate as desired. Software and/or data may be permanently or temporarily embodied in a certain type of machine, component, physical device, virtual equipment, computer storage medium or device, or transmitted signal wave, to be interpreted by a processing device or to provide a command or data to the processing device. Software may be distributed on a computer system connected by a network and may be stored or executed in a distributed fashion. Software and data may be stored in one or more computer-readable recording devices. Software may be read from another computer-readable medium, such as a data storage device, into a main memory or from another device into the main memory through a communication interface. A software instruction stored in the main memory may have a processor perform processes or operations to be described below in detail. Alternatively, processes corresponding to the principle of the disclosure may be performed by using a fixed line circuit instead of a software instruction or by using the fixed line circuit in combination with the software instruction. Thus, embodiments corresponding to the principle of the disclosure are not limited to a certain combination of a hardware circuit with software.

Terms used in the present application are only for describing particular embodiments and are not intended to limit the disclosure. A singular expression may include a plural expression, unless an apparently different meaning is indicated in the context. With respect to the present application, it will be further understood that the terms "comprises" or "comprising" used herein specify the presence of stated features, integers, steps, operations, members, components, and/or groups thereof, but do not preclude the presence or addition of one or more other features, integers, steps, operations, members, components, and/or groups thereof. Although terms such as "first," "second," etc. may be used herein to describe various elements, the elements shall not be limited by these terms. These terms are used only for distinguishing one element from another element.

A "learning model" described in the disclosure may include all forms of algorithms or methods that are used to learn or understand a certain pattern or structure from data. That is, the learning model may include not only a machine learning model, such as a regression model, a decision-making tree, a random forest, a support vector machine, K closest neighbors, naive bayes, a clustering algorithm, etc., but also a deep learning model, such as a neural network, a convolution neural network, a recurrent neural network, a transformer-based neural network, generative adversarial networks (GANs), an auto-encoder, etc. The "learning model" may indicate a learnt parameter or weight set that is used to predict or classify outputs with respect to certain inputs, and this model may be trained by supervised learning, unsupervised learning, semi-supervised learning, reinforcement learning, etc. Also, this may include not only a single model, but also various learning methods and structures, such as an ensemble model, a multimodal model, a model through transfer learning, etc. Such a learning model may be pre-trained in a computer device which is different from a computer device predicting an output with respect to an input and may be used in another computer device.

A learning model according to an embodiment may include at least one model with respect to object classification, object detection, and position estimation.

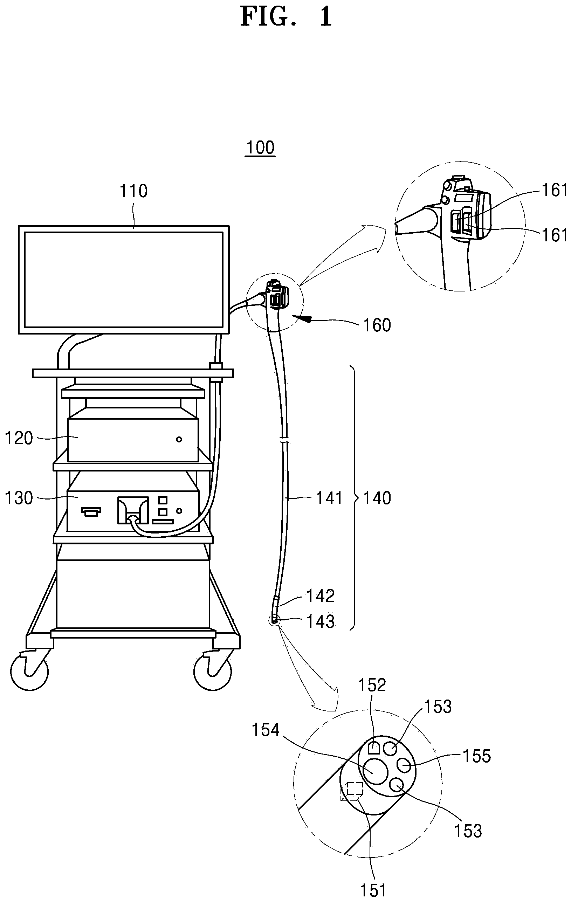

FIG. 1 schematically illustrates an endoscopic device 100 according to an embodiment.

Referring to FIG. 1, the endoscopic device 100 according to an embodiment may be a flexible endoscope, particularly, a gastrointestinal endoscope. The endoscopic device 100 may include a component configured to acquire a medical image of the interior of the digestive tract and a component configured to insert a tool to perform a treatment or procedure while viewing the medical image.

The endoscopic device 100 may include an output unit 110, a controller 120, a driver 130, a scope 140, and a manipulator 160.

The output unit 110 may include a display configured to display a medical image. The output unit 110 may include a display module which is capable of outputting visualized information or realizing a touch screen, such as a liquid crystal display (LCD), a thin-film transistor-LCD (TFT LCD), an organic light-emitting diode (OLED), a flexible display, a three-dimensional (3D) display, etc., and may support image expression, enlargement, and reduction functions, etc. Also, the output unit 110 may allow a user to manipulate an image through a touch screen function and obtain necessary information. The output unit 110 may display a medical image acquired by the scope 140 or a medical image processed by the controller 120.

The driver 130 may provide a driving force necessary for a process of inserting the scope 140 into a human body or moving the scope 140 in the human body. For example, the driver 130 may include a plurality of motors connected to wires in the scope 140 and a tension adjusting portion configured to adjust a tension of the wires. The driver 130 may control the scope 140 in various directions by controlling a driving force of each of the plurality of motors. In detail, the driver 130 may adjust the tension of the wires by controlling the driving force of each of the plurality of motors and may adjust a rotation angle of a front-end portion 143 by bending a curved portion 142.

The scope 140 may include an insertion portion 141, the curved portion 142, and the front-end portion 143. The insertion portion 141 may be inserted into a human body and may be steered by the curved portion 142 and may move to an internal organ.

The curved portion 142 may be connected to the insertion portion 141 and may adjust an insertion direction of the scope 140 with respect to an internal human body. The curved portion 142 may adjust a rotation angle according to a user's command or a control signal of the controller 120.

The front-end portion 143 may be positioned at a front end of the scope 140 and may perform various operations according to a user's command or a control signal of the controller 120. The front-end portion 143 may include an image sensor 151, a nozzle 152, an illumination device 153, a lens 154, and a walking channel 155.

The image sensor 151 may capture an image of the endoscopic device 100. For example, the image sensor 151 may include a complementary metal-oxide-semiconductor (CMOS) sensor or a charge-coupled device (CCD) sensor.

The nozzle 152 may clean the lens 154 by spraying a solution, a drug solution, etc. Also, the nozzle 152 may inject a drug solution necessary for a biopsy or a treatment into an inner portion of the human body.

The illuminator 153 may irradiate a light source for the image sensor 151 to capture an image with constant illuminance. Information about the brightness of the illuminator 153 may be pre-stored in the controller 120.

The lens 154 may focus light so that the image sensor 151 may appropriately capture an image. The lens 154 may include a wide-angle or zoom function.

The walking channel 155 may indicate a channel for transporting an additional instrument or a sampling device into the human body.

The manipulator 160 may indicate a user interface for actually manipulating the endoscope. The manipulator 160 may include a curved steering portion 161 and various buttons, dials, and levers for controlling various functions of the endoscope. A user may input a user's command based on the components provided in the manipulator 160.

The curved steering portion 161 may be used to adjust the curved portion 142. The curved steering portion 161 may be realized in the form of a rotating knob, a joystick, and a lever and may be rotated or moved by a user to steer a direction of the curved portion 142. The curved steering portion 161 may receive torque feedback from the driver 130. For example, the torque feedback may be a physical signal copying a force generated when the front-end portion 143 contacts an internal organ of a human body or based on a control signal of the driver 130 based on a pre-trained model.

The controller 120 may control general operation of the endoscopic device 100 and may perform operations of the endoscopic device 100 according to an embodiment. The controller 120 may control a motion of the scope 140 through the driver 130 connected to the scope 140. The controller 120 may perform various control operations to photograph an internal digestive organ through the scope 140. The controller 120 may perform various processing operations on a medical image acquired through the scope 140.

The controller 120 according to an embodiment may acquire an image of an upper gastrointestinal tract from the image sensor 151, detect, based on a pre-trained model, at least one body part from the image, calculate relative position information between the at least one body part and the front-end portion 143, generate, based on the identified at least one body part and the relative pose information, a control signal for capturing an image, and transmit a control signal to the driver 130. The relative position information may indicate steering information for a movement of the front-end portion 143 from a first position, which is a current position of the front-end portion 143, to a second position. For example, by using the relative position information, the controller 120 may calculate a target rotation angle by which the front-end portion 143 is to be steered toward a body part which is apart from the front-end portion 143 by an x and y coordinate on an image. Also, the controller 120 may control the driver 130 by generating a control signal according to the target rotation angle and, according to the controlling, may adjust pitch and yaw of the front-end portion 143 to steer the front-end portion 143 toward the body part which is apart from the front-end portion 143 by the x and y coordinate on the image.

The controller 120 according to an embodiment may acquire an image of an upper gastrointestinal tract from the image sensor 151, identify, based on a pre-trained model, at least one body part from the image, calculate relative pose information between the at least one body part and the front-end portion 143, generate, based on the identified at least one body part and the relative pose information, a control signal for capturing an image, and transmit a control signal to the driver 130. The relative pose information may indicate information for a change of the front-end portion 143 from a first pose, which is a current pose of the front-end portion 143, to a second pose, for effectively photographing the identified at least one body part. A pose may include a position and a direction of an object in a space. For example, the position may be represented as an x, y, and z coordinate in a coordinate system, and the direction may be represented as pitch (roll around an x-axis), yaw (roll around a y-axis), and roll (roll around a z-axis).

The controller 120 may include a central processing unit (CPU), random-access memory (RAM), read-only memory (ROM), a system bus, etc. The controller 120 may be realized as a single CPU or a plurality of CPUs (or digital signal processors (DSPs) or a system on chip (SoC)). According to an embodiment, the controller 120 may be realized as a DSP configured to process a digital signal, a microprocessor, or a time controller (TCON). However, the controller 120 is not limited thereto and may include or defined by one or more from among a CPU, a micro-controller unit (MCU), a micro-processing unit (MPU), a controller, an application processor (AP), a communication processor (CP), and an advanced reduced-instruction-set-computer (RISC) machine (ARM) processor. Also, the controller 120 may be realized as an SoC, large scale integration (LSI), or an FPGA, in which processing algorithms are embedded. Furthermore, the controller 120 may also include a neural processing unit (NPU), a graphics processing unit (GPU), and a tensor processing unit (TPU).

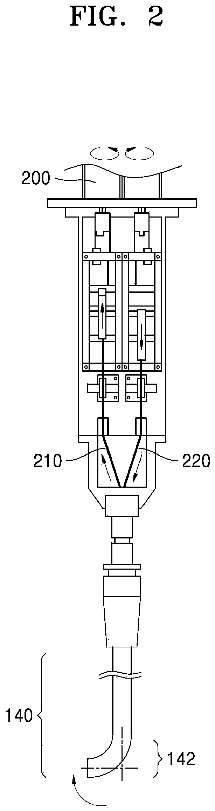

FIG. 2 is a view for describing a process of controlling the curved portion 142 by the driver 130 and wires, according to an embodiment. For convenience of explanation, an operation of controlling a direction of the curved portion 142 by using two motors 200 is illustrated, and it would be obvious for one of ordinary skill in the art that the two motors 200 may be extended to a plurality of motors and may be used to adjust tensions of the wires and adjust a rotation angle of the front-end portion 143.

Referring to FIG. 2, the endoscopic device 100 may include the motor 200 included in the driver 130, a first wire 210, and a second wire 220. The endoscopic device 100 may control the motor 200 to increase a tension of the first wire 210 and decrease a tension of the second wire 220 in order to bend the curved portion 142 to one side. Also, the endoscopic device 100 may control the motor 200 to decrease the tension of the first wire 210 and increase the tension of the second wire 220 in order to bend the curved portion 142 to the other side.

Based on this method, the endoscopic device 100 may adjust a rotation angle of the front-end portion 143.

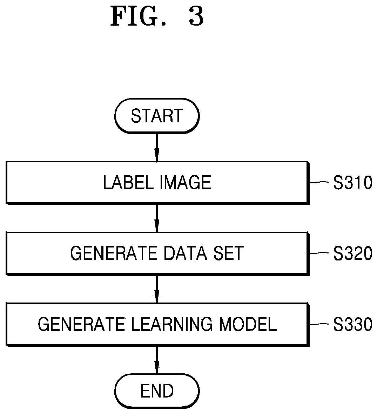

FIG. 3 is a flowchart of an operation of generating a learning model with respect to object detection, according to an embodiment. The learning model in FIG. 3 may correspond to a pre-trained model. For convenience of explanation, it is described that operations are learnt by an additional computer device. However, it is obvious for one of ordinary skill in the art that the operations may be performed by the endoscopic device 100 or an additional computer device.

Referring to FIG. 3, the computer device may tag or label a certain body part by examining an endoscopic image in operation S310. Based on a user input, etc., the computer device may label upper gastrointestinal tract parts on an image by using a bounding box. For example, the upper gastrointestinal tract may include at least one of the oral cavity, the pharynx, the esophagus, the stomach, and the duodenum. Also, the endoscopic image may include luminal images of the oral cavity, the pharynx, the esophagus, the stomach, and the duodenum.

The computer device according to an embodiment may generate a data set including the image on which the labels are designated, in operation S320. The data set may include various illumination conditions, sight angles, states of body parts, etc.

The computer device according to an embodiment may train a neural network model by using the generated data set in operation S330. The computer device may select the neural network model and train the model based on the data set. For example, the model may include an object detection model including a convolution neural network.

A computer device according to another embodiment may generate a learning model with respect to object classification. The learning model with respect to the object classification may be configured to identify an object, an image of which is to be captured.

The computer device according to another embodiment may tag or label a certain body part by examining an endoscopic image in operation S310. Based on a user input, etc., the computer device may label upper gastrointestinal tract parts on the image. The upper gastrointestinal tract parts may indicate body parts, images of which are to be captured.

The computer device according to an embodiment may generate a data set including the image on which the labels are designated, in operation S320. The data set may include various illumination conditions, sight angles, states of body parts, etc.

The computer device according to an embodiment may train a neural network model by using the generated data set in operation S330. The computer device may select the neural network model and train the model based on the data set. For example, the model may include an object classification model including a convolution neural network.

A computer device according to another embodiment may generate a learning model by using an endoscopic image and control history information corresponding to a clinical skill of a user. The control history information may indicate control history information used to observe the endoscopic image and adjust a direction, an angle, a depth, etc. of an endoscope. The computer device according to another embodiment may acquire the endoscopic image and the control history information with respect to the upper gastrointestinal tract in operation S310. For example, the upper gastrointestinal tract may include at least one of the oral cavity, the pharynx, the esophagus, the stomach, and the duodenum. Also, the endoscopic image may include luminal images of the oral cavity, the pharynx, the esophagus, the stomach, and the duodenum.

The computer device according to another embodiment may generate a data set including the endoscopic image and the control history information in operation S320.

The computer device according to another embodiment may train a neural network model by using the generated data set in operation S330. The computer device may select the neural network model and train the model based on the data set. For example, the model may include a classification model such as a convolutional neural network (CNN) to perform image processing and may include at least one of sequence processing models such as a recurrent neural network (RNN) and a long short-term memory (LSTM) to perform sequence processing on the control history information.

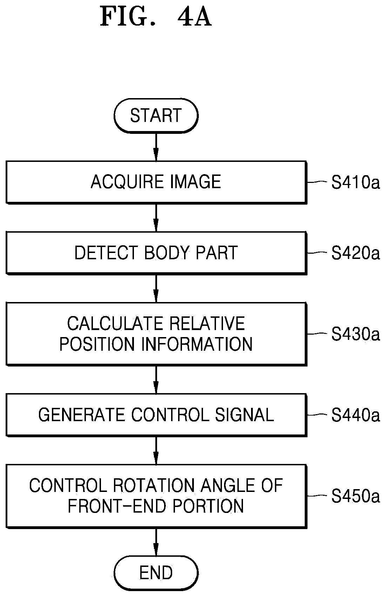

FIG. 4A is a flowchart of an operation of an endoscopic device, according to an embodiment.

Referring to FIG. 4A, the endoscopic device may acquire an image of an upper gastrointestinal tract from an image sensor in operation S410a. For example, a front-end portion of the endoscopic device may be inserted into the upper gastrointestinal tract, and the image obtained by converting an optical signal into an electrical signal may be acquired from the image sensor.

The endoscopic device according to an embodiment may detect, based on a pre-trained model, at least one body part from the image in operation S420a. The endoscopic device may detect the at least one body part, based on the pre-trained model in which the upper gastrointestinal tract parts are labeled as a bounding box, and may identify a position of the at least one body part on the image.

The endoscopic device according to an embodiment may calculate relative position information between the body part and the front-end portion of the endoscopic device in operation S430a. The endoscopic device may calculate the relative position information based on a change of the image according to an angle change of the front-end portion. The endoscopic device may calculate the relative position information based on the disparity and the angle change of the front-end portion.

The endoscopic device according to an embodiment may generate a control signal for performing a steering operation of the front end potion with respect to the position of the body part, based on the relative position information, in operation S440a.

The endoscopic device according to an embodiment may transmit the control signal to a driver in operation S450a. The endoscopic device according to an embodiment may adjust a tension of at least one wire and control a rotation angle of the front-end portion to steer the front-end portion toward the detected body part in operation S450a.

As a detailed example, the endoscopic device according to an embodiment may acquire a first image at a first point and may acquire a second image at a second point, which is a position rotationally moved by a certain distance of $\Delta\theta$. When it is assumed that the disparity between the first image and the second image is $\Delta L$. , $\theta_{target}$, which is for steering the front-end portion, which is apart, by $L_{target}$, from a central coordinate of the bounding box of the detected body part, toward the detected body part, may be represented by Equation 1.

[Equation 1]

$$\theta_{target} = \frac{\Delta\theta}{\Delta L} L_{target}$$

$\Delta\theta$ may be calculated from encoder information of the driver, and $\Delta L$ may be calculated from the disparity between the images, and thus, a relationship formula between a target distance on the image and a target rotation angle by the driver with respect to the front-end portion may be derived.

The Equation 1 as described above may be represented by a coordinate system. That is, the rotation of the front-end portion with respect to a direction in which the front-end portion is inserted into the body part is roll, and thus, the endoscopic device may steer the front-end portion toward the body part, which is apart from the front-end portion by an x and y coordinate on the image, by controlling pitch and yaw via the driver.

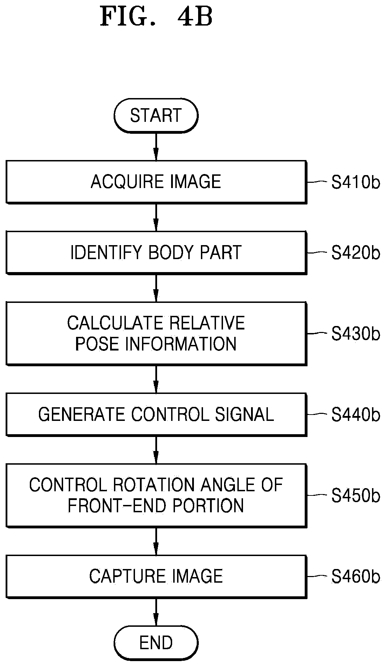

FIG. 4B is a flowchart of an operation of an endoscopic device, according to another embodiment.

Referring to FIG. 4B, the endoscopic device may acquire an image of an upper gastrointestinal tract from an image sensor in operation S410b. For example, a front-end portion of the endoscopic device may be inserted into the upper gastrointestinal tract, and the image obtained by converting an optical signal into an electrical signal may be acquired from the image sensor.

The endoscopic device according to another embodiment may identify, based on a pre-trained model, at least one body part from the image in operation S420b. The endoscopic device may classify the body parts based on the pre-trained model in which the upper gastrointestinal tract is labelled and may identify the at least one body part in the image.

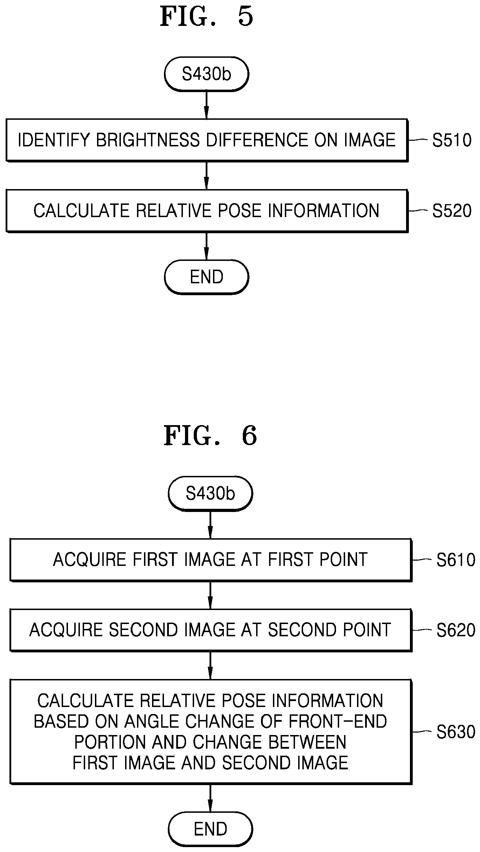

The endoscopic device according to another embodiment may calculate relative pose information including a distance between the body part and the front-end portion of the endoscopic device, etc. in operation S430b. For example, the endoscopic device may calculate the relative pose information based on the brightness of an illuminator, an image change according to an angle change of the front-end portion, and a movement trajectory of the front-end portion. An operation, performed by the endoscopic device, of calculating the relative pose information may correspond to FIGS. 5, 6, and 7.

The endoscopic device according to another embodiment may generate a control signal for capturing an image, based on the identified body part and the relative pose information, in operation S440b. The control signal may include a signal for adjusting the front-end portion of the endoscope.

The endoscopic device according to another embodiment may transmit the control signal to a driver in operation S450b. The endoscopic device according to an embodiment may adjust a tension of at least one wire to correspond to at least one predetermined capturing point with respect to the identified body part and may control a rotation angle of the front-end portion in operation S450b.

The endoscopic device according to another embodiment may capture an image, based on the identified body part and the relative pose information, in operation S460b. The endoscopic device may capture the image when the front-end portion is positioned at the at least one predetermined capturing point with respect to the identified body part.

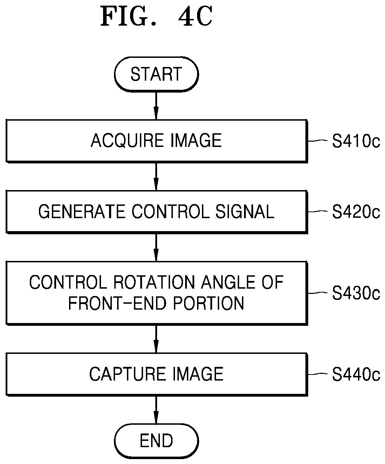

FIG. 4C is a flowchart of an operation of an endoscopic device, according to another embodiment.

Referring to FIG. 4C, the endoscopic device may acquire an image of an upper gastrointestinal tract from an image sensor in operation S410c. For example, a front-end portion of the endoscopic device may be inserted into the upper gastrointestinal tract, and the image obtained by converting an optical signal into an electrical signal may be acquired from the image sensor.

The endoscopic device according to another embodiment may generate a control signal for capturing an image, based on a model pre-trained by using an endoscopic image and control history information corresponding to a clinical skill of a user, in operation S420c. The endoscopic device may generate the control signal by inputting the endoscopic image into a learning model including a classification model and a sequence processing model. The control signal may include a signal for adjusting the front-end portion of the endoscope.

The endoscopic device according to another embodiment may transmit the control signal to a driver in operation S430c. The endoscopic device may adjust a tension of at least one wire to correspond to at least one predetermined capturing point with respect to the identified body part and may control a rotation angle of the front-end portion in operation S430c.

The endoscopic device according to another embodiment may capture an image, based on the identified body part and the relative pose information, in operation S440c. The endoscopic device may capture the image when the front-end portion is positioned at the at least one predetermined capturing point with respect to the identified body part.

FIG. 5 is a flowchart of an operation, performed by an endoscopic device, of calculating relative pose information, according to an embodiment. The operation of the endoscopic device in FIG. 5 may correspond to operation S430b of FIG. 4B. The relative pose information may indicate information for a change of the front-end portion 143 from a first pose, which is a current pose of the front-end portion 143, to a second pose, for effectively photographing the identified at least one body part.

Referring to FIG. 5, the endoscopic device may identify a brightness difference on an image, based on an acquired image and brightness information of an illuminator, in operation S510. The endoscopic device may analyze a difference between stored brightness information of the illuminator and brightness information of the image captured by an image sensor and the illuminator. The endoscopic device may identify a brightness pattern generated according to the characteristic of an internal organ and an illumination intensity and direction.

The endoscopic device according to an embodiment may calculate the relative pose information based on the identified brightness difference in operation S520. The endoscopic device may estimate, based on the identified brightness difference, a distance between the front-end portion and a certain body part at the current pose of the front-end portion, and based on the estimated distance, a central coordinate of the image, and a coordinate of the identified body part, may calculate position information for movement of the front-end portion and direction information for the image sensor provided in the front-end portion to capture an image. The relative pose information may also include a path and a direction in which the front-end portion is moved through the internal human body and a necessary angle change of the front-end portion.

The endoscopic device according to an embodiment may calculate the relative pose information not only by using the brightness difference, but also by projecting light of a certain pattern based on structured light and using a change of this pattern. For example, the endoscopic device may estimate the distance based on the change of the pattern and based on the estimated distance, the central coordinate of the image, and the coordinate of the identified body part, may calculate the position information for the movement of the front-end portion and the direction information for the image sensor provided in the front-end portion to capture an image.

The endoscopic device according to an embodiment may calculate a distance from the front-end portion to a lesion by analyzing a difference in pixel between two images by using a plurality of image sensors.

FIG. 6 is a flowchart of an operation, performed by an endoscopic device, of calculating relative pose information, according to another embodiment. The operation of the endoscopic device in FIG. 6 may correspond to operation S430b of FIG. 4B.

Referring to FIG. 6, the endoscopic device may acquire a first image at a first point in operation S610.

The endoscopic device according to another embodiment may acquire a second image at a second point in operation S620.

The endoscopic device according to another embodiment may calculate the relative pose information based on an angle change of a front-end portion and a pixel change on a screen during a movement of the endoscopic device from the first point to the second point, in operation S630.

For example, the endoscopic device may analyze an input image by using a convolution operation. The endoscopic device may calculate a position of an identified body part as a coordinate in the form of a bounding box, based on a pre-trained model which is trained by extracting features of an input image through various convolution layers. The coordinate may include a left coordinate, a top coordinate, a right coordinate, and a bottom coordinate, and the output coordinate of the bounding box may be converted into a central coordinate of the bounding box after a post-processing process.

The central coordinate of the bounding box may be represented by Equation 2.

[Equation 2]

$$\hat{x} = \frac{l + r}{2}$$
$$\hat{y} = \frac{t + b}{2}$$

x^ indicates a central coordinate of an x axis taking an average value of a left boundary and a right boundary, and y^ indicates a central coordinate of a y axis taking an average value of a top boundary and a bottom boundary.

The endoscopic device may calculate, based on encoder information, an angle change $\Delta\theta$ of the front-end portion during the movement of the endoscopic device from the first point to the second point and may calculate a distance between the identified body part and the front-end portion by using a pixel change $\Delta L$ of an image.

This calculation may be represented by Equation 3.

[Equation 3]

$$\hat{d} = \frac{R\,\Delta\theta}{\Delta L}f = \frac{\Delta B}{\Delta L}f$$

d^ indicates a distance between the lesion and the front-end portion, R indicates a rotational radius of the front-end portion, $\Delta\theta$ indicates an angle change of the front-end portion of the endoscope, $f$ indicates a focal distance, $\Delta B$ indicates a displacement of an image sensor, and $\Delta L$ indicates a pixel change on a screen, that is, a disparity of the same object on two images. Based on a calculated rotational angle, the endoscopic device may calculate a target angle at which the front-end portion of the endoscope has to be moved, by using multi-traces. The endoscopic device may generate a control signal by analyzing a difference between the calculated target movement angle and a current angle of the front-end portion.

The operation of the endoscopic device as described above may be realized not only through a single image sensor, but also through stereo vision based on a plurality of image sensors. $\Delta B$ may correspond to a distance between the image sensors.

The endoscopic device may estimate the distance and based on the estimated distance, a central coordinate of the image, and a coordinate of the identified body part, may calculate position information for movement of the front-end portion and direction information for the image sensor provided in the front-end portion to capture an image.

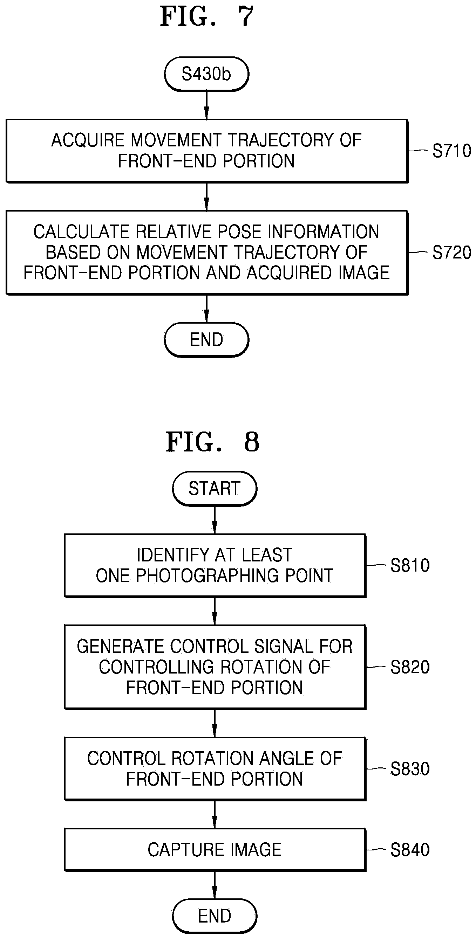

FIG. 7 is a flowchart of an operation, performed by an endoscopic device, of calculating relative pose information, according to another embodiment. The operation of the endoscopic device in FIG. 7 may correspond to operation S430b of FIG. 4B.

Referring to FIG. 7, the endoscopic device may acquire movement trajectory data of a front-end portion in operation S710. The movement trajectory data may refer to data indicating movement of the front-end portion in a human body and for example, may include at least one of track data based on sensors, such as a magnetic-field sensor, a gyroscope, and an accelerometer, and an image-based track data based on an image sensor.

The endoscopic device according to another embodiment may calculate the relative pose information between a body part and the front-end portion, based on the movement trajectory of the front-end portion and an acquired image, in operation S720. For example, the endoscopic device may calculate, based on position information based on an encoder value of a motor and a depth-estimation algorithm, the relative pose information for movement of the front-end portion from a first pose which is a current position to a second pose which is a target position.

FIG. 8 is a flowchart of an operation, performed by an endoscopic device, of capturing an image, according to an embodiment. The operation of the endoscopic device of FIG. 8 may correspond to operations S440b to S460b in FIG. 4B and operations S420b to S440b in FIG. 4B.

Referring to FIG. 8, the endoscopic device may identify one or more pre-set photographing points with respect to an identified body part in operation S810. The one or more pre-set photographing points may be pre-defined positions for photographing or treating a body part and may indicate positions that are pre-set by a user according to a clinical skill of the user, etc. or positions at which structural information of the identified body part may be obtained.

In operation S820, the endoscopic device according to an embodiment may generate, based on the one or more photographing points and relative pose information, a control signal for controlling a rotation of the front-end portion. In detail, the endoscopic device may calculate the relative pose information with respect to the body part and the endoscopic device and based on the calculated relative pose information and a distance and a direction between the one or more pre-set photographing points, may generate the control signal for allowing the front-end portion to be positioned at one or more photographing points.

The endoscopic device according to an embodiment may transmit the control signal to a driver, and the driver may adjust a tension of at least one wire, based on the control signal, to bend a curved portion to control a rotation angle of the front-end portion, in operation S830.

The endoscopic device according to an embodiment may photograph an image based on an image sensor at each of the one or more photographing points, when the front-end portion is arranged at each of the one or more photographing points, in operation S840.

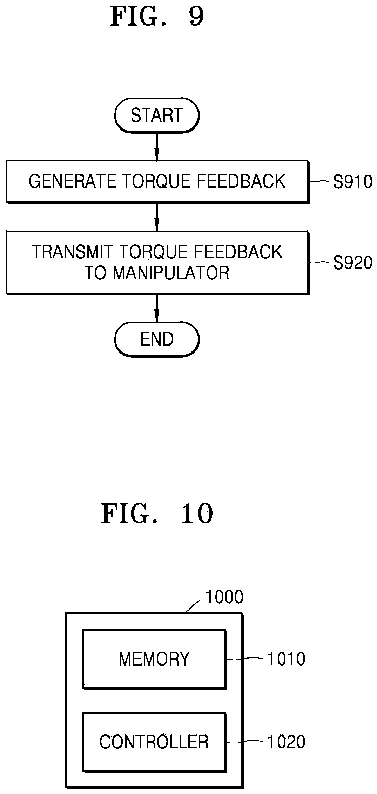

FIG. 9 is a flowchart of an operation of an endoscopic device with respect to torque feedback, according to an embodiment. The torque feedback may be a physical signal copying a force generated when a front-end portion contacts an internal organ of a human body or based on a control signal of a driver based on a pre-trained model.

Referring to FIG. 9, in operation S910, the endoscopic device may generate the torque feedback related to the movement of the front-end portion. The torque feedback may be generated based on a control signal with respect to photographing of an image. The torque feedback may be generated based on information regarding in which direction and by which degree the front-end portion of the endoscopic device has to be rotated, and a movement distance and a size of the torque feedback may have a positive correlation.

In operation S920, the endoscopic device may transmit the torque feedback to a manipulator. For example, the endoscopic device may control the front-end portion according to a rotation angle by which the front-end portion has to be controlled for photographing an image, and a curved steering portion may transmit the torque feedback in a direction in which the front-end portion is steered, thereby giving feedback to a user.

FIG. 10 is a block diagram of a computer device according to an embodiment.

The computer device may include a memory 1010 and a processor 1020. The computer device may be a separate device from an endoscopic device or may be included in a controller. The computer device may execute one or more sets of instructions configured to perform arbitrary one or more of the methods described in this specification.

The memory 1010 may store a set of instructions including an instruction related to a system and an instruction related to a user interface, the instructions being configured to perform arbitrary one or more functions of the methods described in this specification. The memory 1010 may temporarily or permanently store a basic program, an application program, and data such as setting information for operation of a device. The memory 1010 may include a permanent mass storage device, such as RAM, ROM, and a disk drive, but the disclosure is not limited thereto. These software components may be loaded from an additional computer-readable recording medium, which is different from the memory 1010, by using a drive mechanism. The additional computer-readable recording medium may include a computer-readable recording medium, such as a floppy drive, a disk, a tape, a digital versatile disc (DVD)/compact disc (CD)-ROM drive, a memory card, etc. According to an embodiment, the software components may be loaded in the memory 1010 through a communicator, rather than from the computer-readable recording medium. Also, the memory 1010 may provide stored data in response to a request by the processor 1020. The memory 1010 according to an embodiment may store setting information.

The processor 1020 may control general operations of the computer device. Also, the processor 1020 may be configured to process commands by performing basic arithmetic, logic, and input and output operations. The command may be provided to the processor 1020 by the memory 1010. For example, the processor 1020 may be configured to execute the command received according to a program code stored in a recording device such as the memory 1010. For example, the processor 1020 may control the device to perform the operations according to various embodiments described above.

The processor 1020 according to an embodiment may tag or label a certain body part by examining an endoscopic image. Based on a user input, etc., the computer device may label or classify upper gastrointestinal tract parts on an image as a bounding box. For example, the upper gastrointestinal tract may include at least one of the oral cavity, the pharynx, the esophagus, the stomach, and the duodenum.

The processor 1020 according to an embodiment may generate a data set including the image on which the labels are designated. The data set may include various illumination conditions, sight angles, states of body parts, etc.

The processor 1020 according to an embodiment may train a neural network model by using the generated data set. The computer device may select the neural network model and train the model based on the data set. For example, the model may include at least one of an object detection model and an object classification model and may include a convolution neural network.

The processor 1020 according to another embodiment may generate a learning model by using the endoscopic image and control history information corresponding to a clinical skill of a user. The control history information may indicate control history information used to observe the endoscopic image and adjust a direction, an angle, a depth, etc. of an endoscope. The processor 1020 may acquire the endoscopic image and the control history information with respect to an upper gastrointestinal tract. For example, the upper gastrointestinal tract may include at least one of the oral cavity, the pharynx, the esophagus, the stomach, and the duodenum.

The processor 1020 according to another embodiment may generate a data set including the endoscopic image and the control history information.

The processor 1020 according to another embodiment may train a neural network model by using the generated data set. The computer device may select the neural network model and train the model based on the data set. For example, the model may include a classification model such as a CNN to perform image processing and may include at least one of sequence processing models such as an RNN and an LSTM to perform sequence processing on the control history information.

The embodiments are described above according to limited examples and drawings. However, one of ordinary skill in the art could make various modifications and alterations from the disclosure above. For example, even when the described techniques are performed in a different order from the described method and/or the components of the described system, structure, device, circuit, etc. are combined or assembled in a different form from the described method or replaced or substituted by other components or equivalents, appropriate outcomes may be achieved.

Therefore, other realized examples, other embodiments, and the equivalents of the claims shall be included in the claims described below.

[DESCRIPTION OF REFERENCE NUMERALS]

100: ENDOSCOPIC DEVICE
110: OUTPUT UNIT
120: CONTROLLER
130: DRIVER
140: SCOPE
141: INSERTION PORTION
142: CURVED PORTION
143: FRONT-END PORTION
150: SCOPE
151: IMAGE SENSOR
152: NOZZLE
153: ILLUMINATION DEVICE
154: LENS
155: WALKING CHANNEL
160: MANIPULATOR
161: CURVED STEERING PORTION
200: MOTOR
210: FIRST WIRE
220: SECOND WIRE
1010: MEMORY
1020: PROCESSOR

**Claims**

1. A method of controlling an endoscopic device, the method comprising:

   acquiring an image of an upper gastrointestinal tract from an image sensor;
   detecting at least one first body part from the image, based on a pre-trained model;
   calculating relative position information between the detected at least one first body part and a front-end portion of the endoscopic device;
   generating, based on the relative position information, a first control signal for steering the front-end portion to correspond to the at least one first body part; and
   transmitting the first control signal to a driver.

2. The method of claim 1, wherein the acquiring of the image of the upper gastrointestinal tract comprises:

   acquiring a first image at a first point; and
   acquiring a second image at a second point, and
   the calculating of the relative position information comprises calculating a target rotation angle of the front-end portion of the endoscopic device based on i) an angle change of the front-end portion of the endoscopic device between the first point and the second point and ii) a change between the first image and the second image.

3. The method of claim 1, further comprising:

   identifying at least one second body part from the image based on the pre-trained model;
   calculating relative pose information between the identified at least one second body part and the front-end portion;
   generating, based on the relative pose information, a second control signal with respect to photographing of the identified at least one second body part; and
transmitting the second control signal to the driver.

4. The method of claim 3, wherein the calculating of the relative pose information comprises:

identifying, based on the acquired image and brightness information with respect to an illuminator, a brightness difference on the image;
estimating, based on the identified brightness difference, a distance between the front-end portion and the at least one second body part; and
calculating the relative pose information based on the estimated distance.

5. The method of claim 3, wherein the acquiring of the image of the upper gastrointestinal tract comprises:

acquiring a first image at a first point; and
acquiring a second image at a second point, and
the calculating of the relative pose information comprises:

estimating a distance between the front-end portion and the at least one second body part based on i) an angle change of the front-end portion between the first point and the second point and ii) a change between the first image and the second image; and
calculating the relative pose information.

6. An endoscopic device comprising:

a front-end portion including an image sensor;
a driver configured to control a rotation angle of the front-end portion; and
a controller configured to:

detect, based on a pre-trained model at least one first body part from an image;
calculate relative position information between the detected at least one first body part and the front-end portion;
generate, based on the relative position information, a first control signal for steering the front-end portion to correspond to the at least one first body part; and
transmit the first control signal to the driver.

7. The endoscopic device of claim 6, wherein the controller is further configured to acquire a first image at a first point and a second image at a second point and calculate, based on i) an angle change of the front-end portion between the first point and the second point and ii) a change between the first image and the second image, a target rotation angle of the front-end portion.

8. The endoscopic device of claim 6, wherein the controller is further configured to:

identify, based on the pre-trained model, at least one second body part from the image;
calculate relative pose information between the identified at least one second body part and the front-end portion;
generate, based on the relative pose information, a second control signal with respect to photographing of the at least one second body part; and
transmit the second control signal to the driver.

9. The endoscopic device of claim 8, wherein the controller is further configured to:

identify, based on the acquired image and brightness information with respect to an illuminator, a brightness difference on the image;
estimate, based on the identified brightness difference, a distance between the front-end portion and the at least one second body part; and
calculate the relative pose information based on the estimated distance.

10. The endoscopic device of claim 8, wherein the controller is further configured to:

acquire a first image at a first point and a second image at a second point; and
based on i) an angle change of the front-end portion between the first point and the second point and ii) a change between the first image and the second image, estimate a distance between the front-end portion and the at least one second body part and calculate the relative pose information.

11. The endoscopic device of claim 8, wherein the pre-trained model comprises a classification model and a detection model both trained by using, as a data set, an image labelled with respect to the at least one first body part and the at least one second body part with respect to the upper gastrointestinal tract.

12. The endoscopic device of claim 8, wherein the controller is further configured to:

identify at least one photographing point corresponding to the identified at least one second body part;
generate the second control signal, based on the at least one photographing point and the relative position information; and
photograph an image when a position of the front-end portion corresponds to the at least one photographing point.

13. The endoscopic device of claim 6, further comprising a curved portion connected to the front-end portion, wherein the controller is further configured to control the driver based on the first control signal and adjust a tension of at least one wire connected to the driver so as to adjust, based on a curve of the curved portion, the rotation angle of the front-end portion.

14. The endoscopic device of claim 6, further comprising a manipulator including a curved steering portion, wherein the controller is further configured to generate torque feedback and transmit the torque feedback to the curved steering portion via the driver.

15. The endoscopic device of claim 14, wherein the torque feedback has a positive correlation with a target rotation angle for moving the front-end portion according to the first control signal with respect to the photographing of the image.

# FIG. 1

# FIG. 2

# FIG. 3

```
        ┌─────────┐
        │  START  │
        └─────────┘
             │
             ▼
┌───────────────────────────┐
│        LABEL IMAGE        │──── S310
└───────────────────────────┘
             │
             ▼
┌───────────────────────────┐
│     GENERATE DATA SET     │──── S320
└───────────────────────────┘
             │
             ▼
┌───────────────────────────┐
│  GENERATE LEARNING MODEL  │──── S330
└───────────────────────────┘
             │
             ▼
        ┌─────────┐
        │   END   │
        └─────────┘
```

# FIG. 4A

```
         ( START )
             │
             ▼
┌─────────────────────────┐
│     ACQUIRE IMAGE       │── S410a
└─────────────────────────┘
             │
             ▼
┌─────────────────────────┐
│    DETECT BODY PART     │── S420a
└─────────────────────────┘
             │
             ▼
┌─────────────────────────┐
│   CALCULATE RELATIVE    │── S430a
│   POSITION INFORMATION  │
└─────────────────────────┘
             │
             ▼
┌─────────────────────────┐
│  GENERATE CONTROL SIGNAL│── S440a
└─────────────────────────┘
             │
             ▼
┌─────────────────────────┐
│ CONTROL ROTATION ANGLE OF│── S450a
│    FRONT-END PORTION    │
└─────────────────────────┘
             │
             ▼
          (  END  )
```

# FIG. 4B

START

ACQUIRE IMAGE — S410b

IDENTIFY BODY PART — S420b

CALCULATE RELATIVE
POSE INFORMATION — S430b

GENERATE CONTROL SIGNAL — S440b

CONTROL ROTATION ANGLE OF
FRONT-END PORTION — S450b

CAPTURE IMAGE — S460b

END

# FIG. 4C

```
        ┌─────────┐
        │  START  │
        └────┬────┘
             ▼
┌──────────────────────────────┐
│        ACQUIRE IMAGE         │──── S410c
└──────────────┬───────────────┘
               ▼
┌──────────────────────────────┐
│    GENERATE CONTROL SIGNAL   │──── S420c
└──────────────┬───────────────┘
               ▼
┌──────────────────────────────┐
│   CONTROL ROTATION ANGLE OF  │──── S430c
│       FRONT-END PORTION      │
└──────────────┬───────────────┘
               ▼
┌──────────────────────────────┐
│        CAPTURE IMAGE         │──── S440c
└──────────────┬───────────────┘
               ▼
        ┌─────────┐
        │   END   │
        └─────────┘
```

# FIG. 5

```
            ( S430b )
                │
                ▼
┌────────────────────────────────────────┐
│ IDENTIFY BRIGHTNESS DIFFERENCE ON IMAGE │── S510
└────────────────────────────────────────┘
                │
                ▼
┌────────────────────────────────────────┐
│    CALCULATE RELATIVE POSE INFORMATION  │── S520
└────────────────────────────────────────┘
                │
                ▼
            (  END  )
```

# FIG. 6

```
            ( S430b )
                │
                ▼
┌────────────────────────────────────────┐
│     ACQUIRE FIRST IMAGE AT FIRST POINT  │── S610
└────────────────────────────────────────┘
                │
                ▼
┌────────────────────────────────────────┐
│   ACQUIRE SECOND IMAGE AT SECOND POINT  │── S620
└────────────────────────────────────────┘
                │
                ▼
┌────────────────────────────────────────┐
│    CALCULATE RELATIVE POSE INFORMATION  │
│   BASED ON ANGLE CHANGE OF FRONT-END    │── S630
│     PORTION AND CHANGE BETWEEN          │
│     FIRST IMAGE AND SECOND IMAGE        │
└────────────────────────────────────────┘
                │
                ▼
            (  END  )
```

# FIG. 7

S430b

ACQUIRE MOVEMENT TRAJECTORY OF
FRONT-END PORTION — S710

CALCULATE RELATIVE POSE INFORMATION
BASED ON MOVEMENT TRAJECTORY OF
FRONT-END PORTION AND ACQUIRED IMAGE — S720

END

# FIG. 8

START

IDENTIFY AT LEAST
ONE PHOTOGRAPHING POINT — S810

GENERATE CONTROL SIGNAL FOR
CONTROLLING ROTATION OF
FRONT-END PORTION — S820

CONTROL ROTATION ANGLE OF
FRONT-END PORTION — S830

CAPTURE IMAGE — S840

END

# FIG. 9

```
        ( START )
            |
            v
+---------------------------+
|  GENERATE TORQUE FEEDBACK |——S910
+---------------------------+
            |
            v
+---------------------------+
|  TRANSMIT TORQUE FEEDBACK |——S920
|       TO MANIPULATOR      |
+---------------------------+
            |
            v
        ( END )
```

# FIG. 10

```
              1000
        +------------------+
        | +--------------+ |
        | |   MEMORY     |——1010
        | +--------------+ |
        |                  |
        | +--------------+ |
        | |  CONTROLLER  |——1020
        | +--------------+ |
        +------------------+
```

Europäisches
Patentamt

European
Patent Office

Office européen
des brevets

## EUROPEAN SEARCH REPORT

**Application Number**

EP 24 15 9282

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/296281 A1 (YEUNG CHUNG-KWONG [CN] ET AL) 18 October 2018 (2018-10-18) * paragraphs [0011], [0091] - [0094], [0101], [0123], [0131], [0136], [0139] * | 1-15 | INV. A61B1/00 A61B1/005 |
| | ----- | | |
| X | KR 102 553 961 B1 (MEDINTECH INC [KR]) 10 July 2023 (2023-07-10) * paragraph [0042] * | 1 | |
| | ----- | | |
| X | US 2023/148847 A1 (GONO KAZUHIRO [JP] ET AL) 18 May 2023 (2023-05-18) * paragraphs [0213], [0217] * | 1 | |
| | ----- | | |

**TECHNICAL FIELDS SEARCHED (IPC)**

A61B

The present search report has been drawn up for all claims

| Place of search | Date of completion of the search | Examiner |
|---|---|---|
| Munich | 31 July 2024 | Hemb, Björn |

EPO FORM 1503 03.82 (P04C01)

## ANNEX TO THE EUROPEAN SEARCH REPORT
## ON EUROPEAN PATENT APPLICATION NO.

EP 24 15 9282

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2024

| Patent document cited in search report | Publication date | Patent family member(s) | Publication date |
|---|---|---|---|
| US 2018296281 A1 | 18-10-2018 | CN 108685560 A | 23-10-2018 |
| | | US 2018296281 A1 | 18-10-2018 |
| | | WO 2018188466 A1 | 18-10-2018 |
| KR 102553961 B1 | 10-07-2023 | NONE | |
| US 2023148847 A1 | 18-05-2023 | NONE | |

EPO FORM P0459

For more details about this annex : see Official Journal of the European Patent Office, No. 12/82